Europäisches Patentamt

**European Patent Office**

**Office européen des brevets**

(11) Numéro de publication: **0 260 188**
**A1**

# DEMANDE DE BREVET EUROPEEN

(21) Numéro de dépôt: **87401988.8**

(22) Date de dépôt: **04.09.87**

(51) Int. Cl.⁴: **G 01 N 33/546**
**G 01 N 33/545**

(30) Priorité: **12.09.86 FR 8612794**

(43) Date de publication de la demande:
**16.03.88 Bulletin 88/11**

(84) Etats contractants désignés:
**AT BE CH DE ES GB GR IT LI LU NL SE**

(71) Demandeur: **I.B.A.L. Société à Responsabilité Limitée dite:**
**7, Avenue Joliot Curie Z.I. de Périgny**
**F-17000 La Rochelle (FR)**

(72) Inventeur: **Benajam, Alain**
**36, av. Karl Marx**
**F-93000 Bobigny (FR)**

(74) Mandataire: **Bonnetat, Christian et al**
**Cabinet PROPI Conseils 23 rue de Léningrad**
**F-75008 Paris (FR)**

(54) **Dispositif pour l'analyse d'un liquide biologique par mise en oeuvre de réactions d'immuno-adhérence, et paillettes destinées à être utilisées dans ce dispositif.**

(57) - La présente invention concerne un dispositif pour l'analyse d'un liquide biologique par mise en oeuvre de réactions d'immuno-adhérence, comportant au moins un support (3) en matière synthétique, pouvant être mis en contact avec un liquide biologique (5), où sont fixés des antigènes (4), respectivement des anticorps, susceptibles d'engendrer une réaction spécifique d'immuno-adhérence en présence d'anti-corps (6), respectivement d'antigènes, correspondants dans le liquide biologique (5).

- Le dispositif est rendu remarquable, selon l'invention, par des paillettes (7) en matière synthétique, portant des éléments immunologiquement actifs (8), destinées à être mises en contact avec le support et pouvant adhérer audit support (3) par l'intermédiaire desdits éléments (8).

- L'invention s'applique à l'analyse de liquides biologiques.

FIG.4

## Description

Dispositif pour l'analyse d'un liquide biologique par mise en oeuvre de réactions d'immuno-adhérence, et paillettes destinées à être utilisées dans ce dispositif.

La présente invention a pour objet un dispositif pour l'analyse d'un liquide biologique par mise en oeuvre de réactions d'immuno-adhérence, ainsi que des paillettes detinées à être utilisées dans ce dispositif.

En médecine, les praticiens doivent souvent déterminer la nature exacte des antigènes portés par une cellule ou particule, ainsi que celle des anticorps ou des antigènes viraux contenus dans un liquide biologique, comme par exemple du sang, de l'urine, du sperme ou du liquide céphalo-rachidien.

Une telle nécessité est particulièrement évidente dans le domaine des transfusions sanguines où il convient de s'assurer que le sang des donneurs est bien compatible avec celui du receveur, ou ne risque pas de lui transmettre des maladies virales.

On peut distinguer différentes sortes d'antigènes : par exemple, les antigènes érithrocytaires qui sont des motifs antigéniques de la membrane des globules rouges ou les antigènes viraux portés par les virus. De façon plus générale, on désignera par "antigène" toute substance ayant une activité antigénique, c'est-à-dire toute substance susceptible d'entraîner la formation d'anticorps correspondants dans le milieu biologique dans lequel elle est introduite. Quant aux anticorps, ce sont des protéines ayant des structures moléculaires très proches les unes des autres et se distinguant seulement les uns des autres par leurs sites de liaison susceptibles de réagir spécifiquement avec l'antigène qui provoque la formation d'un anticorps particulier.

Ainsi, dans le cas où l'on effectuerait une transfusion sanguine alors qu'une incompatibilité existe entre les sangs du donneur et du receveur, la fixation d'anticorps présents dans un sang sur les motifs antigéniques portés par les globules rouges de l'autre sang peut agglutiner ceux-ci, les détruire ou du moins les fragiliser gravement. Par exemple, si les globules rouges d'un patient sont du type "A", ce qui signifie que des antigènes "A" sont portés par les globules rouges, le sérum de son sang aura des anticorps "anti-B", c'est-à-dire des anticorps qui réagiront avec des globules rouges "B". Si un tel patient reçoit du sang "B", une réaction immunologique se produira entre les anticorps "anti-B" du sérum du patient et les antigènes "B" des globules rouges du donneur.

Différents procédés existent, à l'heure actuelle, pour analyser des liquides biologiques, et en particulier pour déterminer la présence dans ces liquides d'antigènes et/ou d'anticorps spécifiques.

On procède en général de la manière suivante :
- on fait réagir un liquide dans lequel on doit révéler la présence éventuelle d'un antigène donné avec un réactif contenant une quantité connue de l'anticorps correspondant, ou on fait réagir un réactif contenant une quantité connue d'un antigène avec un liquide dans lequel on doit déterminer la présence éventuelle de l'anticorps correspondant ;

- on sépare le complexe antigène/anticorps, quand il existe, des composants qui n'ont pas réagi ; et,
- on mesure la quantité d'antigène, ou d'anticorps, qui a réagi.

Pour faciliter la séparation du produit de réaction complexe du mélange réactionnel, on a proposé de fixer le réactif contenant des anticorps (ou des antigènes) sur un substrat polymère solide pour y former une monocouche réactive d'anticorps (ou d'antigènes) dont les sites de liaison sont correctement orientés. Puis, cette couche est mise en contact avec une solution contenant des antigènes (ou des anticorps) potentiellement immunologiquement réactifs.

Mais, tous les procédés actuellement existants sont difficiles à mettre en oeuvre, et nécessitent des appareillages encombrants et coûteux, ainsi qu'un personnel spécialisé.

La présente invention a pour but d'éviter ces inconvénients, et concerne un dispositif pour l'analyse d'un liquide biologique par mise en oeuvre de réactions d'immuno-adhérence qui peut être utilisé facilement, n'importe où et dans n'importe quelle condition.

A cet effet, le dispositif pour l'analyse d'un liquide biologique par mise en oeuvre de réactions d'immuno-adhérence, du type comportant au moins un support en matière synthétique, pouvant être mise en contact avec un liquide biologique, où sont fixés des antigènes, respectivement des anticorps, susceptibles d'engendrer une réaction spécifique immunologique en présence éventuelle d'anticorps, respectivement d'antigènes, correspondants dans le liquide biologique, est rendu remarquable, selon l'invention, par des paillettes en matière synthétique, portant des éléments immunologiquement actifs, destinées à être mises en contact avec ledit support et pouvant adhérer audit support par l'intermédiaire desdits éléments.

Dans un premier cas, les éléments immunologiques actifs des paillettes sont susceptibles d'adhérer aux anticorps, respectivement aux antigènes, éventuellement présents dans le liquide biologique, qui se sont eux-mêmes fixés aux antigènes, respectivement aux anticorps, dudit support si une réaction immunologique a effectivement eu lieu dans le liquide biologique.

Ainsi, dans le cas où une réaction immunologique a lieu dans le liquide biologique, celle-ci est immédiatement détectée grâce auxdites paillettes adhérant audit support. Cette réaction est appelée "immuno-adhérence". En effet, le support étant au contact d'une suspension de paillettes, celles-ci peuvent se fixer sur ledit support portant des antigènes, respectivement des anticorps, si des anticorps, respectivement des antigènes, correspondants du liquide biologique se sont préalablement fixés sur ledit support. Si au moins une paillette s'est fixée sur ledit support, on peut affirmer qu'il y a réaction d'immuno-adhérence et qu'une réaction immunologique a bien eu lieu à l'interface liquide biologique-support. Le dispositif conforme à l'invention permet donc l'analyse qualitative rapide desdits liquides biologiques et, éventuellement, l'analyse quantitative de ceux-ci par dénombrement des paillettes adhérant audit support.

Dans un second cas, les éléments immunologiquement actifs des paillettes sont susceptibles d'adhérer aux antigènes, respectivement aux anticorps, fixés audit support si une réaction immunologique, bloquant les sites actifs des antigènes, respectivement des anticorps, du support, n'a pas eu lieu entre les antigènes, respectivement les anticorps, du support et les anticorps, respectivement les antigènes, du liquide biologique.

Ainsi, si les anticorps ou les antigènes recherchés dans le liquide biologique sont bien présents et se sont fixés à la surface du support, les paillettes ne pourront pas y adhérer, en révélant de ce fait une réaction positive, car les anticorps, ou les antigènes, recherchés auront bloqué les sites actifs des antigènes, ou des anticorps, du support.

En revanche, si ces anticorps, ou antigènes, recherchés ne sont pas présents dans le liquide biologique, les sites de fixation du support resteront disponibles et les paillettes pourront y adhérer en révélant ainsi une réaction négative.

Une telle possibilité est particulièrement intéressante dans le cas où un seul site de fixation est disponible sur l'anticorps, ou l'antigène, recherché dans le liquide biologique, site unique qui est utilisé pour la fixation au support, et que, par conséquent, aucun site ne reste disponible pour la fixation aux paillettes.

La fixation des paillettes est possible du fait que, sur toute la surface de celles-ci destinée à venir au contact dudit support, on peut fixer des éléments immunologiquement actifs qui tous pourront réagir sur le support, directement ou indirectement, ce qui permet d'obtenir un grand nombre de liaisons entre chaque paillette et le support.

En particulier, si la surface dudit support destinée à venir en contact avec le liquide biologique est au moins sensiblement plane, alors la surface des paillettes destinée à venir au contact dudit support et portant lesdits éléments immunologiquement actifs est également au moins sensiblement plane.

En outre, les paillettes ne doivent pas avoir une taille trop importante, ce qui risquerait d'entraîner une rupture des liaisons entre antigènes, anticorps et éléments immunologiquement actifs des paillettes, ni une taille trop faible, ce qui rendrait difficile la détection de leur présence sur le support.

Ainsi, selon d'autres caractéristiques de l'invention, les paillettes présentent une épaisseur comprise entre environ 10 et environ 30 micromètres, et, de préférence, une grande dimension de l'ordre de 50 à 100 micromètres.

Selon encore d'autres caractéristiques de l'invention, les paillettes sont réalisées, au moins en surface, en nitropolystyrène, et peuvent être colorées notamment par des pigments organiques.

Selon d'autres caractéristiques de la présente invention, le support peut être constitué par au moins une partie de paroi d'un récipient contenant ledit liquide biologique, et peut présenter différents emplacements destinés à recevoir, chacun, des antigènes, respectivement des anticorps, ayant une spécificité déterminée.

En outre, l'invention se rapporte également aux paillettes elles-mêmes telles que définies ci-dessus.

Les figures du dessins annexé feront bien comprendre comment l'invention peut être réalisée. Sur ces figures, des références identiques désignent des éléments semblables.

Les figures 1 à 4 sont des vues partielles et schématiques d'un récipient, contenant un liquide biologique, utilisé dans la présente invention, et qui illustrent les différentes étapes relatives à l'emploi du dispositif selon l'invention, dans un premier cas.

La figure 5 est une vue schématique d'un récipient utilisé dans la présente invention.

Les figures 6 à 9 illustrent les différentes étapes relatives à l'emploi du dispositif selon l'invention, dans un second cas.

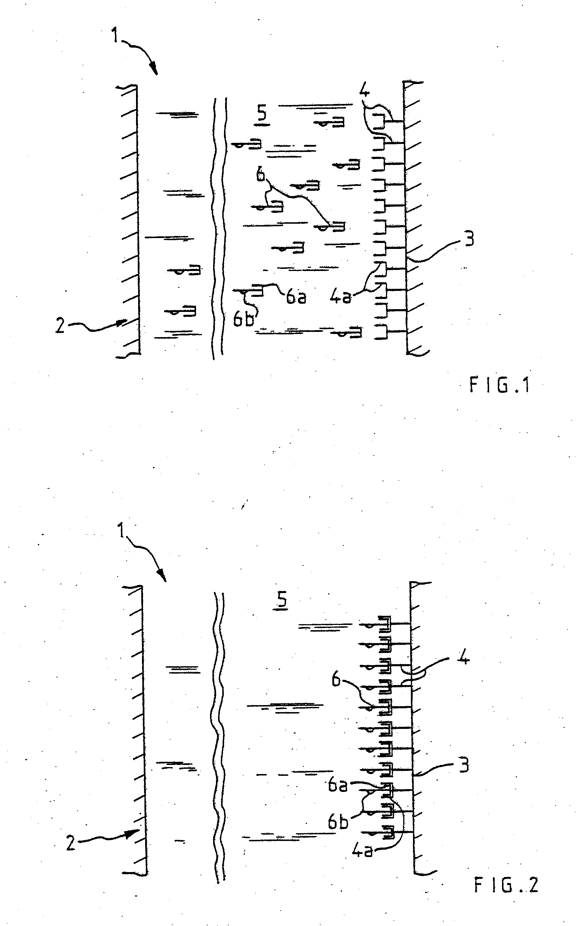

La figure 1 représente schématiquement et partiellement un récipient 1 dont au moins une partie de paroi 2 constitue un support 3, en matière synthétique telle que du polystyrène, pour, par exemple, des antigènes 4 qui y sont fixés, et qui est destiné à contenir un liquide biologique 5 présentant des anticorps 6 spécifiques des antigènes 4 et dont on veut mettre en évidence la présence dans le liquide biologique 5.

Il est bien évident que la représentation des antigènes, des anticorps et autres éléments immunologiquement actifs dans le dessin n'est que symbolique.

Comme indiqué précédemment, quand des antigènes et des anticorps correspondants sont mis en présence les uns des autres, il se produit entre eux une réaction spécifique immunologique qui se traduit par la fixation desdits antigènes auxdits anticorps par l'intermédiaire de sites de liaison 4a, 6a correspondants portés par ceux-ci.

Ainsi, une fois le liquide biologique 5 mis en contact avec le support 3, les anticorps 6 auront tendance à se fixer sur les antigènes 4 du support 3, ce qui est illustré par la figure 2.

Le dispositif de l'invention comporte de plus des paillettes 7 réalisées, au moins en surface, en matière synthétique, telle que du nitropolystyrène, et portant des éléments immunologiquement actifs 8, c'est-à-dire des éléments présentant des sites de liaison 8a correspondants à des sites de liaison 6b portés par les anticorps 6 en plus des sites de liaison 6a servant à la fixation des anticorps 6 sur les antigènes 4.

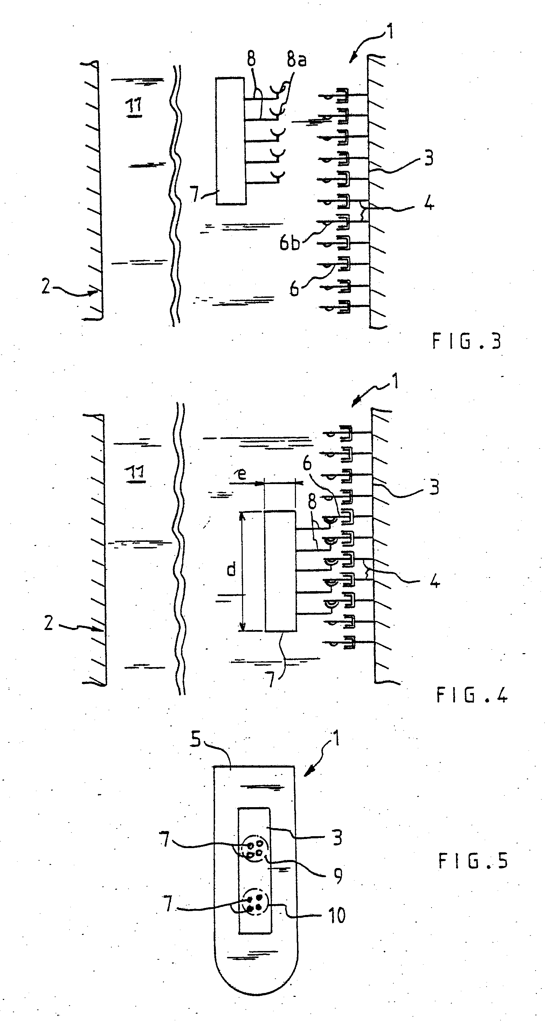

Après lavage, par un liquide physiologique, du support afin d'en éliminer toute substance non spécifiquement fixée, les paillettes 7 suspendues dans un liquide 11, dont une seule est représentée sur la figure 3, sont destinées à être mises en contact avec le support 3 (figure 4) et sont susceptibles d'y adhérer par l'intermédiaire desdits éléments 8 susceptibles de se fixer aux anticorps 6 présents dans le liquide biologique 5 qui se sont eux-mêmes fixés aux antigènes 4.

L'immunoadhérence des paillettes 7 est possible du fait que, sur toute la surface de celles-ci destinée à venir au contact du support 3, on peut fixer des éléments immunologiquement actifs 8 qui tous pourront se fixer au support 3, si une réaction immunologique entre les antigènes 4 et les anticorps 6 a bien eu lieu, ce qui permet d'obtenir un grand nombre de liaisons entre chaque paillette 7 et le support 3.

En particulier, si la surface du support 3 destinée à venir en contact avec le liquide biologique 5 est au moins sensiblement plane, la surface des paillettes 7 destinée à venir au contact du support 3, et portant les éléments 8, sera également au moins sensiblement plane.

Pour permettre aux paillettes 7 de se fixer au support 3 sans risquer la rupture des liaisons entre les paillettes et le support 3, les paillettes 7 ne doivent pas présenter des dimensions trop importantes. En outre, pour permettre une détection aisée de la présence des paillettes 7 sur le support 3, celles-ci ne doivent pas non plus présenter des dimensions trop faibles. De préférence, les paillettes 7 présentent une épaisseur e comprise entre environ 10 et environ 30 micromètres.

Les paillettes 7 peuvent présenter différentes formes : elles peuvent être rondes, ovales, carrées ou rectangulaires, etc...Dans tous les cas, leur grande dimension d est, de préférence, de l'ordre de 50 à 100 micromètres.

De plus, les paillettes 7 peuvent être colorées, notamment par des pigments organiques.

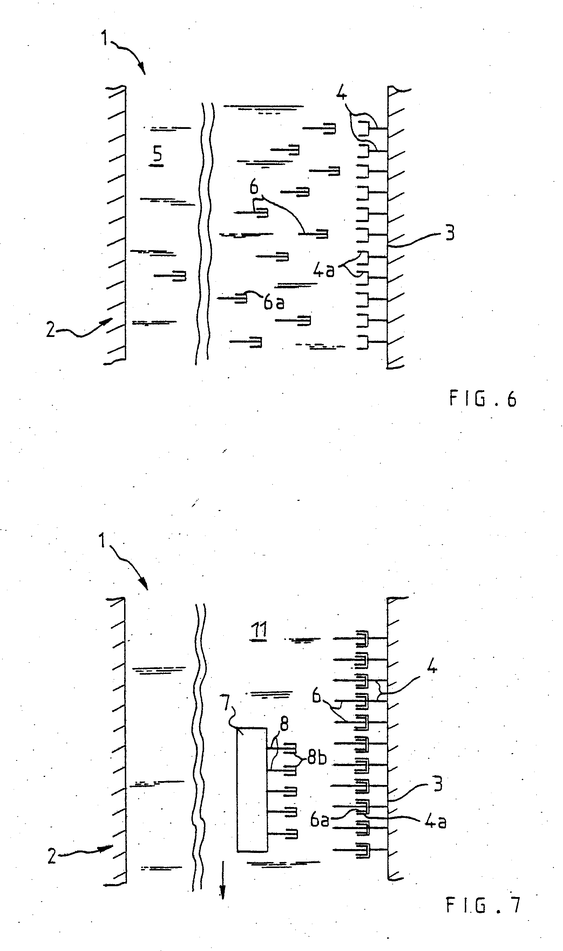

Le support 3 peut présenter différents emplacements 9, 10, montrés agrandis sur la figure 5, destinés, chacun, à recevoir des antigènes, ou éventuellement des anticorps, ayant une spécificité déterminée. On pourra alors utiliser différentes séries de paillettes 7 portant, chacune, des éléments immunologiquement actifs de spécificité déterminée ; chaque série de paillettes, pouvant avoir une coloration propre, étant susceptible d'adhérer sur un emplacement 9, 10 correspondant. Pour mieux mettre en évidence la réaction d'immuno-adhérence, les emplacements 9, 10 peuvent présenter des formes particulières et, éventuellement, différentes les unes des autres, comme par exemple la forme d'une croix grecque, d'un losange, etc.

Enfin, on notera que les "intermédiaires" entre le support et les paillettes, au lieu d'être de simples anticorps ou antigènes, peuvent être également des globules, germes, virus, etc..., portant eux-mêmes des anticorps ou des antigènes susceptibles de se fixer au support, les paillettes pouvant alors elles-mêmes se fixer auxdits globules, germes, virus, etc..., par l'intermédiaire desdits anticorps ou antigènes qu'ils portent.

Les figures 6 à 9 illustrent les différentes étapes relatives à l'emploi du dispositif selon l'invention, dans un second cas.

Le liquide biologique 5 contient, par exemple, des anticorps 6 qui ne présentent cette fois, chacun, qu'un seul site de liaison 6a (figure 6). Les sites de liaison 6a correspondant à ceux 4a des antigènes 4 portés par le support 3, les anticorps 6 vont se fixer sur ces derniers en bloquant ainsi touts les sites actifs des antigènes 4 du support 3 (figure 7). Les paillettes 7 suspendues dans un liquide 11 et mises en contact après lavage du support ne pourront donc pas adhérer au support 3, bien qu'elles présentent des éléments immunologiquement actifs 8 dont les sites de liaison 8b correspondent aux sites 4a des antigènes 4 du support 3, et les paillettes auront tendance à tomber au fond du récipient 1 sous l'action de la gravité, en révélant ainsi une réaction positive.

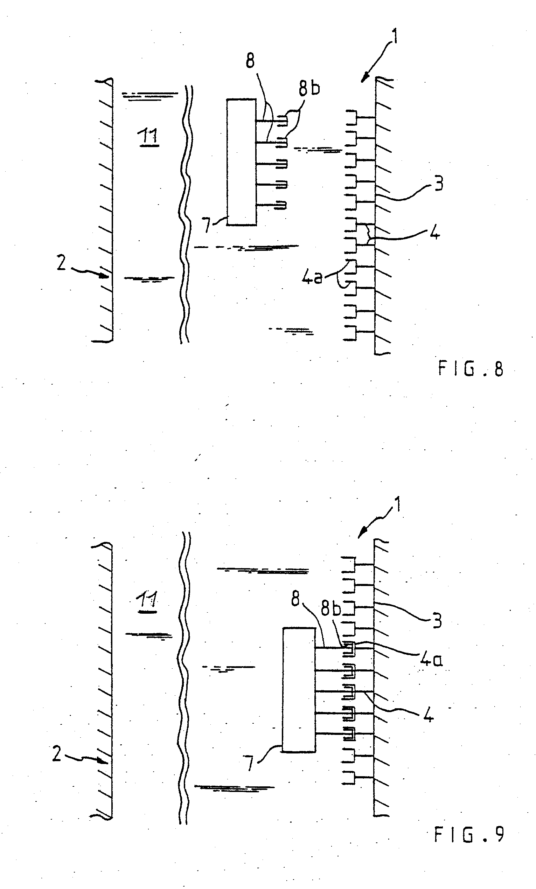

En revanche, en l'absence des anticorps, ou des antigènes, recherchés dans le liquide biologique 5, les paillettes 7, suspendues dans un liquide 11 (figure 8), pourrant adhérer au support 3 (figure 9), en révélant ainsi une réaction négative, puisque les sites de liaison 4a des antigènes 4, ou des anticorps, du support 3 restent libres.

## Revendications

1 - Dispositif pour l'analyse d'un liquide biologique par mise en oeuvre de réactions d'immuno-adhérence, du type comportant au moins un support en matière synthétique, pouvant être mis en contact avec un liquide biologique, où sont fixés des antigènes, respectivement des anticorps, susceptibles d'engendrer une réaction spécifique immunologique en présence éventuelle d'anticorps, respectivement d'antigènes, correspondants dans le liquide biologique, caractérisé par des paillettes (7) en matière synthétique, portant des éléments immunologiquement actifs (8), destinées à être mises en contact avec le support (3) et pouvant adhérer audit support (3) par l'intermédiaire desdits éléments (8).

2 - Dispositif selon la revendication 1, caractérisé en ce que les éléments immunologiquement actifs (8) des paillettes (7) sont susceptibles de se fixer aux anticorps (6), respectivement aux antigènes, éventuellement présents dans le liquide biologique (5), qui se sont eux-mêmes fixés aux antigènes (4), respectivement aux anticorps, dudit support (3) si une réaction immunologique a effectivement eu lieu dans le liquide biologique (5).

3 - Dispositif selon la revendication 1, caractérisé en ce que les éléments immunologiquement actifs (8) des paillettes (7) sont susceptibles de se fixer aux antigènes (4), respectivement aux antiporos, fixés audit support (3) si une réaction immunologique, bloquant les sites actifs (4a) des antigènes (4), respectivement des anticorps, du support (3), n'a pas eu lieu dans le liquide biologique.

4 - Dispositif selon l'une quelconque des revendications 1 à 3, dans lequel la surface dudit support destinée à venir en contact avec le liquide biologique est au moins sensiblement plane, caractérisé en ce que la surface des paillettes (7) destinée à venir au contact dudit support (3) et portant lesdits éléments immunologiquement actifs (8) est au moins sensiblement plane.

5 - Dispositif selon l'une quelconque des revendications 1 à 4,
caractérisé en ce que les paillettes (7) présentent une épaisseur ecomprise entre environ 10 et environ 30 micromètres.

6 - Dispositif selon l'une quelconque des revendications 1 à 5,
caractérisé en ce que les paillettes (7) présentent une grande dimension d de l'ordre de 50 à 100 micromètres.

7 - Dispositif selon l'une quelconque des revendications 1 à 6,
caractérisé en ce que les paillettes (7) sont réalisées, au moins en surface, en nitropolystyrène.

8 - Dispositif selon l'une quelconque des revendications 1 à 7,
caractérisé en ce que les paillettes (7) sont colorées notamment par des pigments organiques.

9 - Dispositif selon l'une quelconque des revendications 1 à 8,
caractérisé en ce que ledit support (3) est constitué par au moins une partie de paroi d'un récipient (2) contenant ledit liquide biologique (5).

10 - Dispositif selon l'une quelconque des revendications 1 à 9,
caractérisé en ce que ledit support (3) présente différents emplacements (9, 10) destinés à recevoir, chacun, des antigènes, respectivement des anticorps, ayant une spécificité déterminée.

11 - Paillettes destinées à être utilisées dans un dispositif pour l'analyse d'un liquide biologique par mise en oeuvre de réactions d'immuno-adhérence, ledit dispositif comportant au moins un support en matière synthétique, où sont fixés des antigènes, respectivement des anticorps, susceptibles d'engendrer une réaction spécifique immunologique en présence éventuelle d'anticorps, respectivement d'antigènes, correspondants dans le liquide biologique,
caractérisées en ce que lesdits paillettes (7) en matière synthétique portant des éléments immunologiquement actifs (8), sont destinées à être mises en contact avec le support (3) et peuvent adhérer audit support (3) par l'intermédiaire desdits éléments (8).

12 - Paillettes selon la revendication 11,
caractérisées en ce que les éléments immunologiquement actifs de celles-ci sont susceptibles de se fixer aux anticorps (6), respectivement aux antigènes, éventuellement présents dans le liquide biologique (5), qui se sont eux-mêmes fixés aux antigènes (4), respectivement aux anticorps, dudit support (3) si une réaction immunologique a effectivement eu lieu dans le liquide biologique (5).

13 - Paillettes selon la revendication 11,
caractérisé en ce que les éléments (8) immunologiquement actifs de celles-ci sont susceptibles de se fixer aux antigènes (4), respectivement aux anticorps, fixés audit support (3) si une réaction immunologique, bloquant les sites actifs (4a) des antigènes (4), respectivement des anticorps, du support (3) n'a pas eu lieu dans le liquide biologique.

14 - Paillettes selon l'une quelconque des revendications 11 à 13,
caractérisées en ce que leur surface destinée à venir au contact dudit support (3) et portant lesdits éléments immunologiquement actifs (8) est au moins sensiblement plane.

15 - Paillettes selon l'une quelconque des revendications 11 à 14,
caractérisées en ce qu'elles présentent une épaisseur comprise entre environ 10 et environ 30 micromètres.

16 - Paillettes selon l'une quelconque des revendications 11 à 15,
caractérisées en ce qu'elles présentent une grande dimension de l'ordre de 50 à 100 micromètres.

17 - Paillettes selon l'une quelconque des revendications 11 à 16,
caractérisées en ce qu'elles sont réalisées, au moins en surface, en nitropolystyrène.

18 - Paillettes selon l'une quelconque des revendications 11 à 17,
caractérisées en ce qu'elles sont colorées notamment par des pigments organiques.

0260188

FIG.1

FIG.2

0260188

FIG.3

FIG.4

FIG.5

0260188

FIG.6

FIG.7

0260188

FIG.8

FIG.9

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| A | EP-A-0 170 746 (COVALENT TECHNOLOGY CORP.) <br> * Page 4, ligne 17 - page 5, ligne 30; page 8, ligne 25 - page 10, ligne 22; page 17, ligne 6 - page 18, ligne 27; revendications * | 1-18 | G 01 N  33/546 <br> G 01 N  33/545 |
| | --- | | |
| A | EP-A-0 070 527 (TORAY INDUSTRIES) <br> * Page 4, ligne 1 - page 5, ligne 7; page 7, ligne 2 - page 9, ligne 15; revendications 1,2,6-9,11-15,18-23 * | 1-18 | |
| | --- | | |
| A | WO-A-8 403 358 (COVALENT TECHNOLOGY CORP.) <br> * Page 8, ligne 24 - page 16, ligne 14; page 23, lignes 5-16; page 25, lignes 20-28; page 58, lignes 5-30 * | 1-18 | DOMAINES TECHNIQUES RECHERCHES (Int. Cl.4) <br><br> G 01 N |
| | --- | | |
| A | FR-A-2 273 281 (PHARMACIA DIAGNOSTICS AB) <br> * Page 2, ligne 19 - page 3, ligne 10; page 4, lignes 15-32; revendications 1,7 * | 1-18 | |
| | ---          -/- | | |

Le présent rapport de recherche a été établi pour toutes les revendications

| Lieu de la recherche | Date d'achèvement de la recherche | Examinateur |
|---|---|---|
| LA HAYE | 22-10-1987 | HITCHEN C.E. |

## DOCUMENTS CONSIDERES COMME PERTINENTS

Page  2

| Catégorie | Citation du document avec indication, en cas de besoin, des parties pertinentes | Revendication concernée | CLASSEMENT DE LA DEMANDE (Int. Cl 4) |
|---|---|---|---|
| A | MACROMOLECULAR CHEMISTRY AND PHYSICS, vol. 187, no. 7, juillet 1986, pages 1603-1610, Basel, CH; R. COUTURIER et al.: "Fixation d'anticorps par liaisons covalents sur le polystyrène. Activation du support et utilisation répétée du système polystyrène anticorps après test immunoenzymatique" * Résumé; page 1605, lignes 19-28 * | 7,17 | |

- - - - -

DOMAINES TECHNIQUES
RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 22-10-1987 | HITCHEN C.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82